# EUROPÄISCHE PATENTSCHRIFT

(11) **EP 0 464 407 B1**
(45) Veröffentlichungstag und Bekanntmachung des Hinweises auf die Patenterteilung: **30.08.1995**
(21) Anmeldenummer: 91109541.2
(22) Anmeldetag: 11.06.1991
(51) Int. Cl.: A61B 19/08

(54) **Medizinisches Operations-Abdecktuch**
Surgical drape
Drap chirurgical

(30) Priorität: 05.07.1990 DE 4021353
(43) Veröffentlichungstag der Anmeldung: 08.01.1992
(73) Patentinhaber: ROTECNO AG, CH-8045 Zürich (CH)
(72) Erfinder: Duncan, John A., Glenrothes KY62QB (GB); Wiedner, Günther, Dr., W-8124 Seeshaupt (DE); Patel, Suresh R., Dr., Dalgety Bay KY115YL (GB)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- EP-A- 0 166 124
- EP-A- 0 269 207
- DE-A- 2 405 124
- DE-U- 8 707 403
- GB-A- 2 221 395
- US-A- 3 538 912
- US-A- 3 955 569
- US-A- 4 027 665

## Beschreibung

Die Erfindung betrifft ein medizinisches Operations-Abdecktuch nach dem Oberbegriff des Patentanspruches 1.

Ein medizinisches Operations-Abdecktuch dieser Art ist der US-A-3 955 569 zu entnehmen. Bei dem bekannten Abdecktuch besteht das dem Patienten zugewandte Gewebe in einem Ausführungsbeispiel aus einem hydrophoben Gewebe, wobei keine genauen Angaben zu der Ausbildung des Gewebes angegeben sind. Das vom Patienten abgewandte Gewebe besteht aus einem hydrophilen Gewebe, das eine flüssigkeitsdichte untere, d.h. dem Patienten zugewandte Oberfläche aufweist.

Ein weiteres medizinisches Abdecktuch ist aus der GB-A-2 221 395 bekannt. Dieses Abdecktuch besteht aus einer Schicht eines hydrophoben bakteriendichten Materials und es werden weitere Tücher aus hydrophilem Material auf dem Abdecktuch auf die dem Patienten abgewandte Seite gelegt, um Operationsflüssigkeiten und Körperflüssigkeiten aufzunehmen.

Bei derartigen medizinischen Operations-Abdecktüchern kommt es auf eine möglichst gute Bakterienbarriere sowie Flusenfreiheit an. Andererseits soll ein solches Abdecktuch aber zumindest in einem gewissen, an die Operationsstelle angrenzenden Bereich Flüssgikeit speichern bzw. absorbieren können.

Das Ziel der Erfindung besteht somit darin, ein medizinisches Operations-Abdecktuch der eingangs genannten Gattung zu schaffen, welches bei einfacher Befestigbarkeit nahe der Operationsstelle einerseits gut bakteriendicht und flusenfrei ist, andererseits aber auf der Außenseite, d.h. der vom Patienten abgewandten Seite ein großes Flüssgikeitsabsorptionsvermögen aufweist, ohne daß die einfache Handhabung beeinträchtigt wird.

Zur Lösung dieser Aufgabe sind die Merkmale des kennzeichnenden Teils des Patentanspruchs 1 vorgesehen.

Auf diese Weise befindet sich auf der Unterseite ein Flüssigkeiten abstoßendes Gewebe, welches außerdem so dicht gewebt sein soll, daß es auch gut bakteriendicht ist. Auf der Außenseite befindet sich dagegen ein hydrophiles Gewebe oder Gewirke, welches bei der Operation von außen kommende Flüssigkeiten absorbieren und damit für die Umgebung unschädlich machen kann. Da beide Gewebe aus endlosen Kunststoffasern bestehen, sind sie extrem flusenarm.

Beide Gewebe sollen insbesondere aus wasch- und sterilisationsfähigen Kunststoffen bestehen. Das weitere Merkmal, wonach das hydrophobe Gewebe am Operationsrand nach oben umgeschlagen und das hydrophile Gewebe nur an dem Umschlagstreifen angenäht ist, und zwar derart, daß das hydrophile Gewebe oder Gewirke im Bereich der Naht nach unten umgeschlagen ist und nur der dadurch gebildete Umschlagstreifen mit dem Umschlagstreifen des hydrophoben Gewebes vernäht ist, ist besonders wichtig, um nun Flüssgikeits- bzw. Bakterienbrücken durch das hydrophobe Gewebe hindurch bzw. zwischen hydrophilem und hydrophobem Gewebe zu vermeiden. Eine besonders bevorzugte Nahtbildung ist durch den Anspruch 2 gekennzeichnet.

Das hydrophile Gewebe oder Gewirke soll so gestaltet und ausgebildet sein z.B. durch eine rippenartig vergrößerte Oberfläche, daß eine von ihm aufgenommene Flüssgikeit bei mechanischer Druckbelastung seitlich ausweicht und nicht durch das hydrophobe Gewebe gepreßt werden kann, welches hierzu entsprechend dicht gewebt und ausgerüstet sein soll.

Von besonderem Vorteil ist es, wenn das hydrophile Gewebe oder Gewirke erst in einem geringen Abstand vom Operationsrand gemäß Anspruch 2 beginnt.

Der Rand wird zweckmäßigerweise gemäß Anspruch 3 ausgebildet, wobei die schweißbare Folie bzw. das aus dieser entstehende Schmelz- oder Klebematerial insbesondere auch eine Versteifungswirkung für den Rand hat.

Von besonderer Bedeutung ist, daß aufgrund der erfindungsgemäßen Ausbildung ein Ausfransen des Abdecktuches im Bereich des Operationsrandes wirksam vermieden ist.

Weiter ist die Ausbildung nach Anspruch 4 zweckmäßig, d.h., daß die beiden Gewebe in dem größten Bereich ihrer Überlappungsfläche nicht miteinander laminiert sind.

Auf diese Weise kann z.B. eine Tasche gemäß Anspruch 10 gebildet sein, die mit saugfähigem oder absorbierendem Material gefüllt werden kann.

An den übrigen Rändern sollen die Gewebe in üblicher Weise gemäß den Ansprüchen 5 und 6 miteinander vernäht und vorzugsweise auch auf sich selbst umgeschlagen sein.

Gemäß Anspruch 7 erstreckt sich das hydrophile Gewebe nur über einen Bruchteil der Fläche des hydrophoben Gewebes, wobei es jedoch bevorzugt ist, wenn der dem Operationsrand zugewandte Bereich des hydrophoben Gewebes bis auf einen freibleibenden Randstreifen nahe dem Operationsrand mit dem hydrophilen Gewebe abgedeckt ist.

Auch der freie Rand des hydrophilen Gewebes soll nach Anspruch 7 auf sich selbst zurückgeschlagen und vernäht sein, um auch hier Flusenfreiheit zu gewährleisten.

Das Ausführungsbeispiel nach Anspruch 8 ist vorteilhaft, weil hierdurch das Tuch permanent antistatisch gemacht werden kann und/oder im Bereich des Operationsrandes problemlos ein Klebeband nach Anspruch 9 angeordnet werden kann, mittels dessen das Tuch am Operationsrand auf die Haut des Patienten aufgeklebt werden kann.

Das erfindungsgemäße Abdecktuch weist also im Bereich des Operationsrandes eine vergleichsweise steife und definierte Kante auf, die ein dichtes Befestigen des Tuches in diesem Bereich an der Haut des Patienten ermöglicht.

Die außerhalb des eigentlichen Operationsfeldes liegenden Ränder des Abdecktuches sind in üblicher Weise umgeschlagen und miteinander vernäht.

Die Erfindung wird im folgenden beispielsweise anhand der Zeichnung beschrieben; in dieser zeigt:
- Fig. 1: einen Schnitt durch ein erfindungsgemäßes Operations-Abdecktuch gemäß Linie I-I in Fig. 3,
- Fig. 2: einen zu Fig. 2 senkrechten Schnitt eines erfindungsgemäßen Operations-Abdecktuches gemäß Linie II-II in Fig. 3 und
- Fig. 3: eine Draufsicht auf ein erfindungsgemäßes Operations-Abdecktuch von außen.

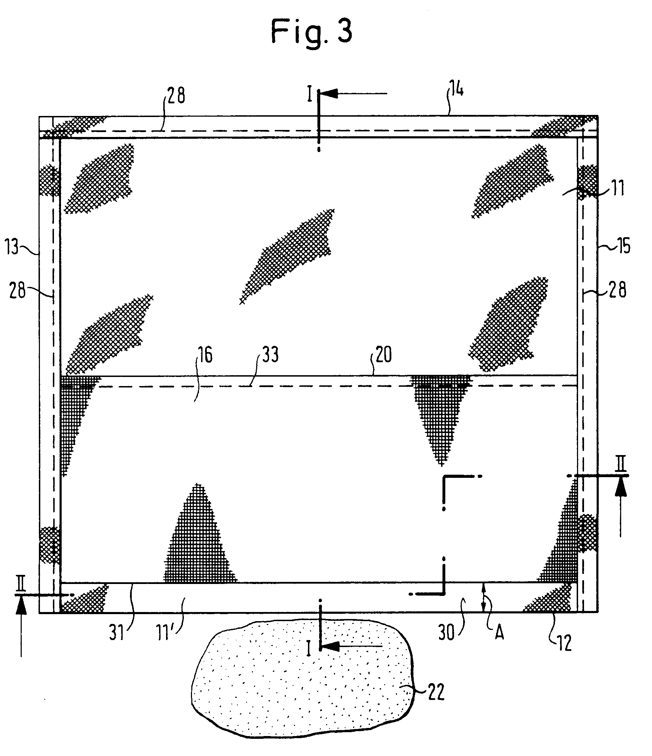

Nach Fig. 3 weist das erfindungsgemäße Operations-Abdecktuch ein rechteckförmiges bakteriendichtes hydrophobes Gewebe 11 aus endlosen Kunststoffäden auf.

Über der in Fig. 3 unteren Hälfte des hydrophoben Gewebes 11 ist ein ebenfalls rechteckförmiges hydrophiles Gewebe 16 angeordnet, das ebenfalls aus endlosen Kunststoffäden gewebt oder gewirkt ist.

Die Seitenränder 13, 15 sowie der Oberrand 14 der Gewebe 11, 16 sind umgeschlagen und durch normale Nähte 28 vernäht, so daß flusenfreie Kanten vorliegen. In ähnlicher Weise ist der obere Rand 20 des hydrophilen Gewebes 16 umgeschlagen und ebenfalls durch eine normale Naht 33 vernäht.

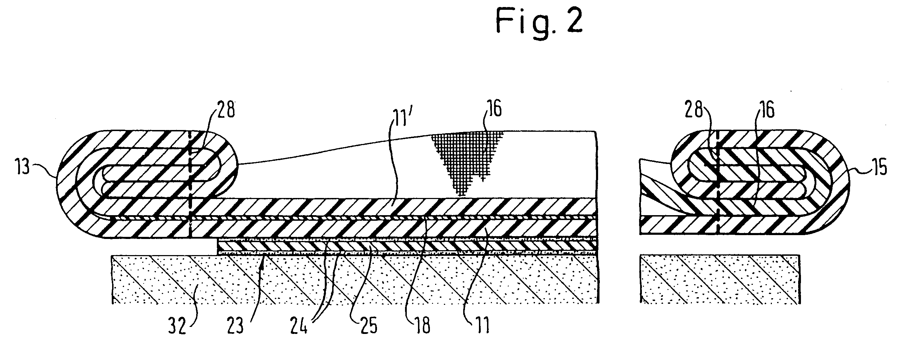

Die Art des Umschlagens und der Vernähung ist auch aus Fig. 2 ersichtlich.

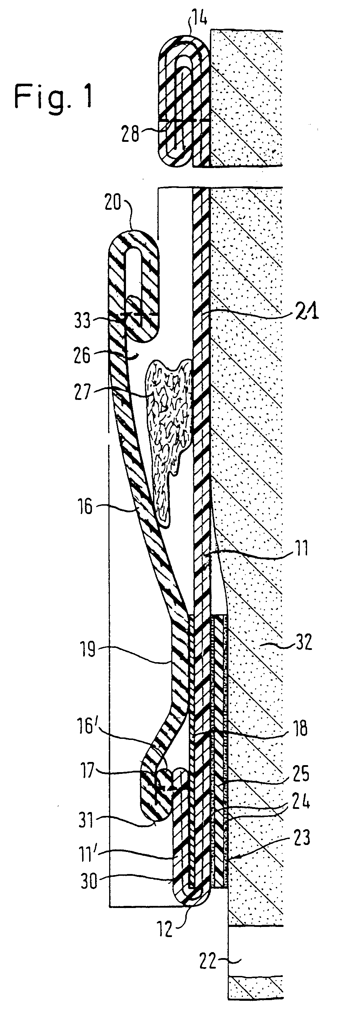

Nach den Fig. 1 und 3 ist das hydrophobe Gewebe 11 im Bereich des der Operationsstelle 22 zugewandten Operationsrandes 12 nach oben auf sich selbst umgeschlagen, so daß ein Umschlagstreifen 11' gebildet wird. Zwischen dem Gewebe 11 und dem Umschlagstreifen 11' ist eine schweißbare Folie 18 (Fig. 1 und 2) angeordnet, welche sich vom Operationsrand 12 weg über den Umschlagstreifen 11' hinaus bis unter einen vergleichsweise schmalen Randbereich 19 des hydrophilen Gewebes 16 erstreckt.

Der Umschlagstreifen 11' ist in der aus Fig. 1 ersichtlichen Weise mit einem nach unten umgeschlagenen Umschlagrand 16' des hydrophilen Gewebes 16 durch eine Naht 17 vernäht, welche sich somit weder durch das Gewebe 11 noch durch das Gewebe 16, sondern nur durch die Umschlagstreifen 11', 16' hindurch erstreckt. Aus diesem Grunde wird in diesem Bereich eine Flüssigkeitsbrücke durch das Tuch hindurch vermieden.

Wesentlich ist, daß zwischen dem Operationsrand 12 und dem dem Operationsrand 12 zugewandten Rand 31 des hydrophilen Gewebes 16 ein schmaler Streifen 30 von etwa 0,5 bis 3 cm verbleibt, der, obwohl er auf der vom Patienten 32 abgewandten Seite sich befindet, hydrophob ist. Der hydrophile Bereich beginnt erst anschließend dort, wo das hydrophile Gewebe 16 vorgesehen ist.

Der Umschlagstreifen 11' und der Randstreifen 19 des hydrophilen Gewebes 16 sind mittels der schweißbaren Folie 18 mit dem darunter liegenden Teil des hydrophoben Gewebes 11 so verschweißt, daß insgesamt ein versteifter Rand 12 gebildet wird, der eine Breite hat, die der Breite des Umschlagstreifens 11' und des anschließenden Randbereiches 19 entspricht.

In diesem Bereich kann problemlos von unten ein mit Klebschichten 24 auf beiden Seiten einer Trägerfolie 25 versehener Doppelklebstreifen 23 angebracht werden, mittels dessen der Randbereich, der an den Operationsrand 12 anschließt, auf die Haut des Patienten 32 aufgeklebt werden kann, wie das aus den Fig. 1 und 2 hervorgeht.

Auf der von dem Operationsrand 12 abgewandten Seite des Randbereiches 19 ist das hydrophile Gewebe 16 außer im Bereich der Seitenränder 13, 15 (Fig. 3) durch die Nähte 28 nicht mit dem hydrophoben Gewebe 11 verbunden, so daß gemäß Fig. 1 eine Tasche 26 gebildet wird, die auf der vom Operationsrand 12 abgewandten Seite durch den Rand 20 des hydrophilen Gewebes 16 begrenzt ist. Im Bereich des Randes 20 ist das hydrophile Gewebe 16 zweimal umgeschlagen und durch eine Naht 33 vernäht, so daß auch in diesem Bereich Flusenfreiheit gewährleistet ist. In das hydrophobpe Gewebe 11 sind gemäß Fig. 1 leitfähige Fäden 21 mit eingewebt, um das Gewebe permanent antistatisch zu machen.

In die Tasche 26 kann gemäß Fig. 1 ein saugfähiges Material wie Baumwolle oder Zellstoff eingebracht werden.

Der Gebrauch des erfindungsgemäßen Operations-Abdecktuchs geht wie folgt vor sich:

Vor Beginn oder auch während der Operation entscheidet der Operateur, ob die Tasche 26 mit flüssigkeitsaufnahmefähigem Material 27 zu füllen ist oder nicht. Gegegebenenfalls wird das saugfähige Material 27 zunächst in die Tasche 26 eingebracht.

In der Regel ist bereits beim Packen, also noch vor dem Sterilisieren des Operations-Sets auf der Unterseite des hydrophobem Gewebes 11 im Bereich des Umschlagstreifens 11' und des Randbereiches 19 ein Klebestreifen 23 gemäß den Fig. 1 und 2 befestigt worden. Mittels dieses Klebstreifens wird dann der betreffende Randbereich des Abdecktuches nahe der Operationsstelle 22 auf die Haut des Patienten 32 aufgeklebt. Da der Randbereich 11', 19 durch die schweißbare Folie 18 vergleichsweise steif ausgebildet ist, kann das Abdecktuch problemlos am Patienten in der gewünschten Position befestigt werden.

Von nun an können von der Haut des Patienten keine Keime oder Bakterien mehr durch das hydrophobe Gewebe hindurchdringen, wodurch die kritische Operationsstelle 22 weitgehend vor Bakterien und Keimen geschützt ist.

Andererseits kann bei der Operation auftretende Flüssigkeit, wie Blut, Wundwasser oder Spülflüssigkeit, die auf die Oberseite des erfindungsgemäßen Abdecktuchs gerät, vom hydrophilen Gewebe 16 sowie dem saugfähigen Material 27 aufgenommen werden. Hierdurch können Reinfektionen durch umherspritzende oder zurückfließende Flüssigkeiten vermieden werden.

Das hydrophobe Gewebe 11 ist beispielsweise ein feinfilamentiertes (Mikrofilament) Polyestergewebe mit einer hydrophoben Ausrüstung, z.B. "Rophobo" (eingetragenes Warenzeichen der Anmelderin).

Das hydrophile Gewebe 16 kann entsprechend, jedoch mit einer hydrophilen Ausrüstung, z.B. "Rophilo" (eingetragenes Warenzeichen der Anmelderin) ausgebildet sein. Es kann auch ein Gewebe oder Gewirk verwendet werden, das durch eine Art Rippenstruktur eine vergrößerte effektive Oberfläche aufweist und sich für ein seitliches Ausweichen der aufgenommenen Flüssigkeit bei mechanischer Druckausübung eignet. Weiter können Gewebe oder Gewirke aus Hohlfasern mit besonderem Vorteil als hydrophiles Gewebe verwendet werden.

Im allgemeinen werden vier erfindungsgemäße Abdecktücher in im wesentlichen rechteckförmiger Anordnung einander überlappend um das in der Mitte befindliche Operataionsfeld herum aufgebracht. Es genügt jedoch in vielen Anwendungsfällen eine geringere Anzahl von Abdecktüchern, die zudem noch an die speziellen Bedingungen einer bestimmten Operation formmäßig angepaßt sein können.

## Patentansprüche

1. Medizinisches Operations-Abdecktuch mit zumindest einer Textilkonstruktion (11), welche einen vorzugsweise geradlinigen, für besondere Anwendungen auch gekrümmten der Operationsstelle (22) zugewandten Operationsrand (12) und weitere, vorzugsweise ebenfalls geradlinige Ränder (13, 14, 15) hat und zweckmäßigerweise rechteckförmig ist, wobei die Textilkonstruktion (11) hydrophob ist und anschließend an den Operationsrand (12) auf dem hydrophoben Gewebe (11) ein hydrophiles Gewebe oder Gewirke (16) angeordnet ist, dadurch gekennzeichnet, daß sowohl das hydrophobe Gewebe (11) als auch das hydrophile Gewebe (16) aus endlosen Kunststoffäden bestehen, und daß das hydrophobe Gewebe (11) am Operationsrand (12) nach oben umgeschlagen und das hydrophile Gewebe (16) nur an dem Umschlagstreifen (11') angenäht ist, und zwar derart, daß das hydrophile Gewebe oder Gewirke (16) im Bereich der Naht (17) nach unten umgeschlagen ist und nur der dadurch gebildete Umschlagstreifen (16') mit dem Umschlagstreifen (11') des hydrophoben Gewebes (11) vernäht ist.

2. Abdecktuch nach Anspruch 1, dadurch gekennzeichnet, daß das hydrophile Gewebe (16) in einem geringen Abstand (A) von insbesondere 0,5 bis 3, insbesondere etwa 2 cm vom Operationsrad beginnt.

3. Abdecktuch nach einem der vorhergehenden Ansprüche,
dadurch **gekennzeichnet,** daß das hydrophobe Gewebe (11) und dessen Umschlagrand (11') durch eine dazwischen angeordnete schweißbare Folie (18) miteinander verbunden, vorzugsweise verschweißt oder auf andere Weise flächig auf Dauer verbunden wird, wobei insbesondere die schweißbare Folie (18) sich vom Operationsrand (12) weg bis unter das hydrophile Gewebe (16) erstreckt und auch dieses in dem dem Operationsrand (12) zugewandten Randbereich (19) mit dem hydrophoben Gewebe (11) verbindet, insbesondere verschweißt.

4. Abdecktuch nach einem der vorhergehenden Ansprüche,
dadurch **gekennzeichnet,**
daß das hydrophile Gewebe (16) zumindest in einem wesentlichen, vom Operationsrand (12) abgewandten Bereich und insbesondere auch an dem vom Operationsrand (12) abgewandten Rand (20) nicht mit dem hydrophoben Gewebe (11) verbunden ist, wobei vorzugsweise die Verbindung zwischen dem hydrophoben und hydrophilen Gewebe (11, 16) nur in einem vergleichsweise schmalen Randbereich (19) unmittelbar anschließend an den Umschlagstreifen (11') des hydrophoben Gewebes (11) vorliegt sowie an den Seitenrändern (13, 15).

5. Abdecktuch nach einem der vorhergehenden Ansprüche,
dadurch **gekennzeichnet,**
daß das hydrophile Gewebe (16) an den Seitenrändern (13, 15) mit dem hydrophoben Gewebe (11) vernäht ist.

6. Abdecktuch nach einem der vorhergehenden Ansprüche,
dadurch **gekennzeichnet,**
daß die Gewebe (11, 16) an den Seitenrändern (13, 15) und dem vom Operationsrand (12) abgewandten Rand (14) umgeschlagen und vernäht sind.

7. Abdecktuch nach einem der vorhergehenden Ansprüche,
dadurch **gekennzeichnet,**
daß sich das hydrophile Gewebe (16) vom Operationsrand (12) nur bis zu einem Bruchteil der Länge der Seitenränder (13, 15), der z.B. 20 bis 60 %, vorzugsweise 30 bis 50 % und insbesondere etwa 40 % beträgt, erstreckt, wobei bevorzugt das hydrophile Gewebe (16) an dem vom Operationsrand (12) abgewandten Rand (20) umgeschlagen und vernäht ist.

8. Abdecktuch nach einem der vorhergehenden Ansprüche,
dadurch **gekennzeichnet,**
daß mindestens eines der beiden Gewebe (11, 16) vorzugsweise das hydrophobe permanent antistatisch ist, insbesondere durch eingewebte leitfähige Fäden (21), und/oder, daß ein an den Operationsrand (12) angrenzender Streifen (11', 19) von vorzugsweise 0,5 bis 5 cm, insbesondere 1 bis 4 cm und zweckmäßigerweise etwa 3 cm Breite, insbesondere durch die schweißbare Folie (18) so verschweißt ist, daß er zwar noch flexibel aber nicht mehr knitterbar ist.

9. Abdecktuch nach einem der vorhergehenden Ansprüche,
dadurch **gekennzeichnet,**
daß nahe dem Operationsrand (12) und parallel zu diesem auf der Unterseite des hydrophoben Gewebes (11) ein mit Klebschichten (24) auf beiden Seiten einer Trägerfolie (25) versehenes Klebeband (23) angebracht ist.

10. Abdecktuch nach einem der vorhergehenden Ansprüche,
dadurch **gekennzeichnet,**
daß die beiden Gewebe eine zum Operationsrand (12) hin geschlossene und am entgegengesetzten Rand (20) offene Tasche (26) bilden, wobei insbesondere in der Tasche (26) ein hydrophiles, insbesondere saugfähiges Material (27) wie Baumwolle oder Zellstoff angeordnet ist.

## Claims

1. Surgical drape comprising at least one textile construction (11), which has a preferably straight edge (12) which faces the site of the operation but which may also be curved for special applications, and further edges (13, 14, 15) which are preferably likewise straight, the drape being expediently rectangular, with the textile construction (11) being hydrophobic and with a hydrophilic fabric or knitted structure (16) being arranged on the hydrophobic fabric (11) adjoining the surgical edge (12), characterised in that both the hydrophobic facric (11) and also the hydrophilic fabric (16) consist of endless synthetic threads; and in that the hydrophobic fabric (11) is turned over upwardly at the surgical edge (12) and the hydrophilic fabric (16) is only sewn to the turned over strip (11'), and indeed in such a way that the hydrophilic fabric or knitted structure (16) is turned over downwardly in the region of the seam (17) and only the thereby formed turned over strip (16') is sewn to the turned over strip (11') of the hydrophobic fabric (11).

2. Drape in accordance with claim 1, characterised in that the hydrophilic fabric (16) starts at a small spacing (A) of in particular 0.5 to 3, in particular approximately 2 cm from the surgical edge (12).

3. Drape in accordance with one of the preceding claims, characterised in that the hydrophobic fabric (11) and its turned over edge (11') are connected together by a weldable foil (18) which is arranged therebetween, and are preferably welded together or permanently areally connected in another manner, with the weldable foil (18) extends away from the surgical edge (12) up to and beneath the hydrophilic fabric (16) and in that the hydrophilic fabric is also connected to the hydrophobic fabric (11), and in particular welded thereto, in the edge region (19) adjacent the surgical edge (12).

4. Drape in accordance with one of the preceding claims, characterised in that the hydrophilic fabric (16) is not connected to the hydrophobic fabric (11), at least in a substantial region remote from the surgical edge (12) and in particular at the edge (20) remote from the surgical edge (12), with the connection between the hydrophobic and the hydrophilic fabrics (11, 16) preferably only being present in a comparatively narrow edge region (19) directly adjoining the turned over strips (11') of the hydrophobic fabric (11) and also at the side edges (13, 15).

5. Drape in accordance with one of the preceding claims, characterised in that the hydrophilic fabric (16) is sewn to the hydrophobic fabric (11) at the side edges (13, 15).

6. Drape in accordance with one of the preceding claims, characterised in that the fabrics (11, 16) are turned over and sewn at the side edges (13, 15) and at the edge remote from the surgical edge (12).

7. Drape in accordance with one of the preceding claims, characterised in that the hydrophilic fabric (16) extends from the surgical edge (12) only up to a fraction of the length of the side edges (13, 15), the fraction amounting for example to 20 to 60%, preferably 30 to 50%, and in particular to approximately 40%, with the hydrophilic fabric (16) preferably being turned over and sewn at the edge (20) remote from the surgical edge (12).

8. Drape in accordance with one of the preceding claims, characterized in that at least one of the two fabrics (11, 16), preferably the hydrophobic fabric, is permanently antistatic, in particular through in-woven conductive threads (21), and/or in that a strip (11', 19) adjoining the surgical edge (12) and of preferably 0.5 to 5 cm, in particular 1 to 4 cm and expediently of approximately 3 cm width is so welded, in particular through the weldable foil (18), that it is indeed still flexible but no longer creasable.

9. Drape in accordance with one of the preceding claims, characterized in that an adhesive strip (23) provided with adhesive layers on both sides of a carrier foil (25) is attached close to the surgical edge (12) and parallel to the latter on the underside of the hydrophobic fabric (11).

10. Drape in accordance with one of the preceding claims, characterized in that the two fabrics form a pocket (26) which is closed towards the surgical edge (12) and open at the opposite edge (20), with a hydrophilic, in particular absorbent material (27) such as cotton or cellular material being arranged in particular in the pocket (26).

## Revendications

1. Drap chirurgical comprenant au moins une structure textile (11) qui comporte un bord opératoire (12), de préférence rectiligne, mais également courbe pour des applications particulières et tourné vers le site opératoire (22), et d'autres bords (13, 14, 15), de préférence également rectilignes, et qui présente, de manière appropriée, une forme rectangulaire, la structure textile (11) étant hydrophobe, un tissu ou un tricot hydrophile (16) étant disposé sur le tissu hydrophobe (11) de manière adjacente au bord opératoire (12), caractérisé en ce que aussi bien le tissu hydrophobe (11) que le tissu hydrophile (16) sont constitués de fils synthétiques continus, et en ce que le tissu hydrophobe (11) est rabattu vers le haut au niveau du bord opératoire (12) et le tissu hydrophile (16) n'est cousin qu'au niveau de la bande de rabat (11') et ce, de façon que le tissu ou le tricot hydrophile (16) soit rabattu vers le bas dans la zone de la couture (17) et que seule la bande de rabat (16') ainsi formée soit cousue avec la bande de rabat (11') du tissu hydrophobe (11).

2. Drap selon la revendication 1, caractérisé en ce que, par rapport au bord opératoire, le tissu hydrophile (16) commence à une faible distance (A) comprise en particulier entre 0,5 et 3 cm et égale en particulier à environ 2 cm.

3. Drap selon l'une des revendications précédentes, caractérisé en ce que le tissu hydrophobe (11) et sa bande de rabat (11') sont reliés entre eux, de préférence par soudage, au moyen d'une feuille soudable (18) disposée entre eux, ou sont solidarisés à plat de manière durable d'une autre manière, la feuille soudable (18) s'étendant en particulier du bord opératoire (12) jusqu'en dessous du tissu hydrophile (16) et reliant également celui-ci, en particulier par soudage, au tissu hydrophobe (11) dans la zone de bord (19) tournée vers le bord opératoire (12).

4. Drap selon l'une des revendications précédentes, caractérisé en ce qu'au moins dans une zone importante opposée au bord opératoire (12) et en particulier aussi sur le bord (20) opposé au bord opératoire (12), le tissu hydrophile (16) n'est pas relié au tissu hydrophobe (11), la liaison entre les tissus hydrophobe et hydrophile (11, 16) n'étant réalisée de préférence que dans une zone de bord comparativement étroite (19) immédiatement adjacente à la bande de rabat (11') du tissu hydrophobe (11) ainsi que sur les bords latéraux (13, 15).

5. Drap selon l'une des revendications précédentes, caractérisé en ce que le tissu hydrophile (16) est cousu avec le tissu hydrophobe (11) au niveau des bords latéraux (13, 15).

6. Drap selon l'une des revendications précédentes, caractérisé en ce que, sur les bords latéraux (13, 15) et sur le bord (14) opposé au bord opératoire (12), les tissus (11, 16) sont rabattus et cousus.

7. Drap selon l'une des revendications précédentes, caractérisé en ce que, à partir du bord opératoire (12), le tissu hydrophile (16) ne s'étend que sur une fraction de la longueur des bords latéraux (13, 15), ladite fraction étant par exemple comprise entre 20 et 60 %, de préférence entre 30 et 50 % et étant égale en particulier à environ 40 %, le tissu hydrophile (16) étant de préférence rabattu et cousu sur le bord (20) opposé au bord opératoire (12).

8. Drap selon l'une des revendications précédentes, caractérisé en ce qu'au moins l'un des deux tissus (11, 16), de préférence celui qui est hydrophobe, est antistatique permanent, en particulier grâce à des fils conducteurs tissés (21) et/ou en ce qu'une bande (11', 19) adjacente au bord opératoire (12) et ayant une largeur de préférence de 0,5 à 5 cm, en particulier de 1 à 4 cm et, de manière appropriée, environ de 3 cm, est soudée en particulier par la feuille soudable (18), de façon à être encore flexible, mais à ne plus être froissable.

9. Drap selon l'une des revendications précédentes, caractérisé en ce qu'un ruban adhésif (23) muni de couches adhésives (24) sut les deux faces d'une feuille porteuse (25) est disposé sur la face inférieure du tissu hydrophobe (11) à proximité du bord opératoire (12) et parallèlement à celui-ci.

10. Drap selon l'une des revendications précédentes, caractérisé en ce que les deux tissus forment une poche (26) fermée en direction du bord opératoire (12) et ouverte sur le bord opposé (20), un matériau hydrophile (27) en particulier absorbant, comme du coton ou de la cellulose, étant placé en particulier dans la poche (26).
